# EUROPÄISCHE PATENTANMELDUNG

(11) **EP 3 906 962 A1**
(43) Veröffentlichungstag der Anmeldung: **10.11.2021**
(21) Anmeldenummer: 20173475.3
(22) Anmeldetag: 07.05.2020
(51) Int. Cl.: A61M 25/10, A61B 17/12

(54) **VORRICHTUNG ZUM VERSCHLIESSEN EINER VENENEINMÜNDUNG BEI DER BEHANDLUNG DER VARIKOSE**

(71) Anmelder: Gefässpraxis Dr. Erpen AG, 3930 Visp (CH)
(72) Erfinder: Erpen, Thomas, 3930 Visp (CH)
(74) Vertreter: AMMANN PATENTANWÄLTE AG

(57) **Zusammenfassung**

Die Vorrichtung zum Verschliessen einer Veneneinmündung bei der Behandlung der Varikose umfasst einen in ein Venensystem einführbaren Katheter (1), an dessen distalen Ende ein aufweitbarer Ballon (5) angeordnet ist, eine Hülle (10), welche umlaufend auf dem Ballon angeordnet ist und mittels diesem aufweitbar ist, wobei das proximale Hüllenende offen ausgestaltet ist, und mindestens ein Haftmittel zum Einwirken auf die aufgeweitete Hülle derart, dass sie zum Verschliessen der Veneneinmündung an einer Venenwand anhaftet.

## Beschreibung

Die Erfindung betrifft eine Vorrichtung zum Verschliessen einer Veneneinmündung bei der Behandlung der Varikose.

Es sind neuere endovenöse Verfahren zur Behandlung der Varikose bekannt, bei welchen Laser, Radiofrequenz oder Kleber zum Verkleben eingesetzt werden. Diese Verfahren sind bei der Behandlung der Vena saphena magna oder der Vena saphena parva problematisch, da die Crosse (auf Englisch "saphenofemoral junction" bzw. "saphenopopliteal junction") nicht genau verschlossen werden kann. Kommt man bei der Behandlung zu nahe an die Crosse, so droht die Gefahr einer Thrombose und Embolie. Ist die Behandlung zu weit weg von der Crosse, kann es zu einem Rezidiv kommen.

Bei der Varikose können auch die Perforansvenen betroffen sein, bei welchen die physiologische Flussrichtung von der oberflächlichen Venen in die tiefen Venen gerichtet ist. Bei der Insuffizienz der Perforansvenen kommt es zu einer Strömungsumkehr in den Perforansvenen, mit Stauungen der oberflächlichen Venen. Die eingangs erwähnten endovenösen Verfahren bieten keine optimale Lösung, um auch das Problem der insuffizienten Perforansvenen behandeln zu können.

Es ist eine Aufgabe der vorliegenden Erfindung, eine Vorrichtung anzugeben, die ein sicheres Verschliessen einer Veneneinmündung bei der Behandlung der Varikose ermöglicht.

Eine Vorrichtung, die diese Aufgabe löst, ist im Patentanspruch 1 angegeben. Die weiteren Patentansprüche geben bevorzugte Ausführungen der Vorrichtung an.

Die Vorrichtung gemäss Anspruch 1 umfasst eine Hülle, welche mittels eines Ballons aufweitbar ist, um eine Veneneinmündung zu verschliessen.

Die Vorrichtung ist für unterschiedliche Anwendungen auslegbar. In einer Anwendung kann die Crosse der Vena saphena magna/parva millimetergenau abgedichtet werden, so dass keine Thrombose ins tiefe Beinvenensystem gelangen kann und kein frühes Varizenrezidiv droht.

In einer anderen Anwendung kann eine Perforansvene sicher abgedichtet werden, so dass der Reflux in ihrem Bereich behoben werden kann, ohne dass durch diese Intervention das tiefe Beinvenensystem gefährdet wird.

Die Vorrichtung ist unterschiedlich ausgestaltbar. Die Hülle der Vorrichtung beispielsweise kann einen röhrenförmigen Mittelteil aufweisen, der entweder nur an einem Hüllenende oder an beiden Hüllenenden offen ausgestaltet ist. Die Hülle ist z.B. sackartig ausgebildet, so dass mit ihr im aufgeweiteten Zustand ein Freiraum in der Vene abgrenzbar ist, der an den einem Ende verschlossen und an dem anderen Ende offen ist. In einer anderen Ausführungsform ist die Hülle durchgehend röhrenförmig ausgebildet, so dass mit ihr im aufgeweiteten Zustand ein Freiraum in der Vene abgrenzbar ist, der an den beiden Enden offen ist.

Weitere spezifische Konstruktionsmerkmale der Vorrichtung und deren Vorteile sind aus folgender Beschreibung und Zeichnungen von Ausführungsbeispielen ersichtlich. Es zeigen
Fig. 1 ein erstes Ausführungsbeispiel einer Vorrichtung in einer Seitenansicht,
Fig. 2 den vorderen Teil der Vorrichtung gemäss Fig. 1 zusammen mit einem Führungsdraht in einer geschnittenen Seitenansicht,
Fig. 3 eine schematische Ansicht der Venen im Bereich der Crosse der Vena saphena magna mit eingeführter Vorrichtung gemäss Fig. 1,
Fig. 4 die Ansicht gemäss Fig. 3 nach der Behandlung, wobei die Hülle geschnitten dargestellt ist,
Fig. 5 eine schematische Ansicht der Venen im Bereich der Crosse der Vena saphena parva nach der Behandlung, wobei die Hülle geschnitten dargestellt ist,
Fig. 6 den vorderen Teil einer Variante der Vorrichtung in einer teilweise geschnittenen Seitenansicht,
Fig. 7 die Vorrichtung gemäss Fig. 6 in einer Vene, wobei Ballon und Hülle im aufgeweiteten Zustand sind,
Fig. 8 den vorderen Teil einer weiteren Variante der Vorrichtung in einer teilweise geschnittenen Seitenansicht,
Fig. 9 die Vorrichtung gemäss Fig. 9 in einer Vene, wobei Ballon und Hülle in einem aufgeweiteten Zustand sind,
Fig. 10 die Ansicht gemäss Fig. 9 nach der Behandlung,
Fig. 11 den vorderen Teil einer anderen weiteren Variante der Vorrichtung in einer Seitenansicht, wobei die Hülle geschnitten dargestellt ist,
Fig. 12 die Vorrichtung gemäss Fig. 11 in einem etwas aufgeweiteten Zustand in einer Vene,
Fig. 13 die Ansicht gemäss Fig. 12 nach der Behandlung,
Fig. 14 eine schematische Ansicht der Venen im Bereich der Crosse der Vena saphena magna, in welche eine Hülle mit Gittergerüsten platziert worden ist, wobei die Hülle geschnitten dargestellt ist,
Fig. 15 den vorderen Teil einer weiteren Variante der Vorrichtung in einer Vene,
Fig. 16 den vorderen Teil einer anderen weiteren Variante der Vorrichtung in einer Vene,
Fig. 17 eine anderen weitere Variante der Vorrichtung ohne Innenkatheter in einer Seitenansicht,
Fig. 18 den Innenkatheter für die Vorrichtung gemäss Fig. 17 in einer Seitenansicht,
Fig. 19 die Vorrichtung gemäss Fig. 17 mit eingeführtem Innenkatheter gemäss Fig. 18 in einer teilweise geschnittenen Seitenansicht,
Fig. 20 ein zweites Ausführungsbeispiel einer Vorrichtung in einer Seitenansicht,
Fig. 21 eine schematische Ansicht der Venen im Bereich einer Perforansvene mit eingeführter Vorrichtung gemäss Fig. 20,
Fig. 22 die Ansicht gemäss Fig. 21 nach der Behandlung, wobei die Hülle geschnitten dargestellt ist,
Fig. 23 eine schematische Ansicht der Venen im Bereich der Crosse der Vena saphena magna nach der Behandlung mit der Vorrichtung gemäss Fig. 20, wobei die Hülle geschnitten dargestellt ist,
Fig. 24 schematisch ein Bein mit einem Teil des Venensystems, und
Fig. 25 eine schematische Ansicht der Venen im Bereich der Crosse der Vena saphena magna nach der Behandlung mit der Vorrichtung gemäss Fig. 20, wobei die Hülle geschnitten dargestellt ist.

In der nachfolgenden Beschreibung werden die Begriffe "proximal" und "distal" aus Sicht des Benutzers der Vorrichtung verwendet. Das proximale Ende ist somit das Ende, welches dem Benutzer zugewandt ist, und das distale Ende ist das dem Benutzer abgewandte Ende.

### Erstes Ausführungsbeispiel

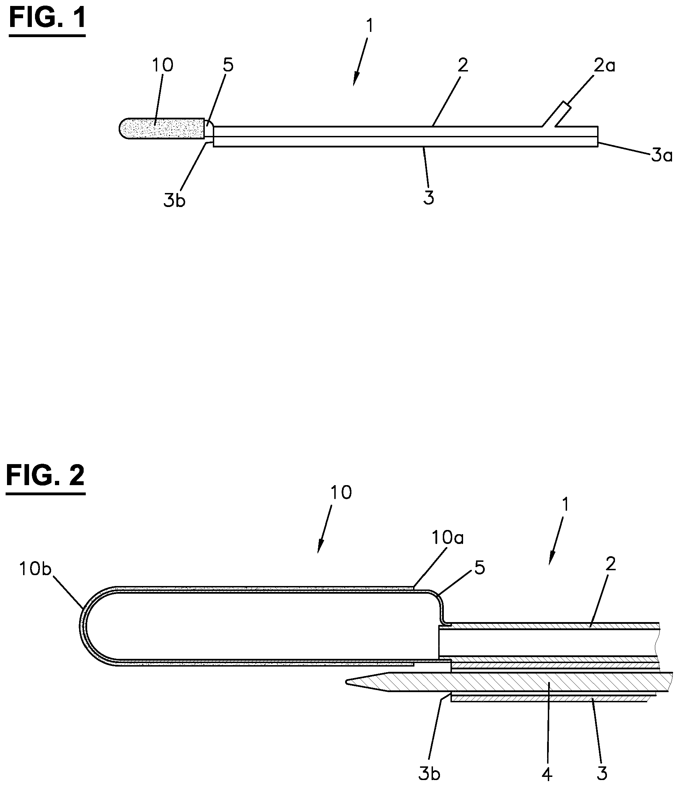

Fig. 1 zeigt einen Katheter 1, der in eine Vene einführbar ist und der am distalen Ende einen aufweitbaren Ballon 5 aufweist. Auf diesem ist eine aufweitbare Hülle 10 angeordnet. Der Katheter 1 weist einen ersten Kanal 2 auf, der einen Anschluss 2a mit dem Ballon 5 fluidisch verbindet, und einen zweiten Kanal 3, der eine Eingangsöffnung 3a mit einer Ausgangsöffnung 3b verbindet.

Am Anschluss 2a ist z.B. eine Spritze oder drgl. anschliessbar, um über den ersten Kanal 2 ein Fluid in den Ballon 5 zu pumpen bzw. aus diesem wieder abzulassen und so ein In- und Deflatieren des Ballons 5 zu ermöglichen. Um einen Patienten durch eine etwaige Undichtigkeit des in die Vene eingeführten Ballons 5 nicht zu gefährden, wird nicht Luft als Fluid zum Inflatieren verwendet, sondern eine Flüssigkeit, beispielsweise eine Natriumchloridlösung.

Wie Fig. 2 zeigt, ist in den zweiten Kanal 3 ein Führungsdraht 4 aufnehmbar. Ballon 5 und Hülle 10 sind im nicht-aufgeweiteten Zustand so in Bezug auf die Ausgangsöffnung 3b angeordnet, dass seitlich genügend Platz zum Vorbeiführen des Führungsdrahtes 4 vorhanden ist.

Die Hülle 10 ist sackartig geformt und somit am proximalen Hüllenende 10a offen und am distalen Hüllenende 10b geschlossen ausgestaltet. Die Länge der Hülle 10 (Distanz zwischen den Enden 10a und 10b) ist im nicht-aufgeweiteten Zustand typischerweise kleiner als 10 cm.

Die Hülle 10 ist wie eine Membrane als dünne Schicht ausgebildet und ist für das Blut, insbesondere das Blutplasma, undurchlässig.

Die Hülle 10 besteht aus einem körperverträglichen Material, z.B. Rinderperikard. Es kann auch ein anderes biologisches oder auch synthetisches Material verwendet werden, das vom Körper toleriert und nicht abgestossen wird. Das Material der Hülle 10, welches im Körper zumindest für eine gewisse Zeit verbleiben soll, ist weich, so dass sie bei Bewegungen nicht schmerzt und/oder scheuert, insbesondere bei Bewegungen im Hüftgelenk, wenn die Hülle 10 in der Nähe eingesetzt wird.

Je nach Anwendungszweck ist das Material der Hülle 10 so gewählt, dass es vom Körper nicht abgebaut wird oder sich nach einer gewissen Zeit auflöst. Im letzteren Fall ist die Zeitdauer bis zur Resorption mindestens so lange, dass bei Verwendung der Vorrichtung zum Verschliessen der Crosse es nicht zur Bildung einer Neocrosse kommt.

Die hier beschriebene Vorrichtung ist vielseitig anwendbar, um eine Veneneinmündung zu verschliessen.

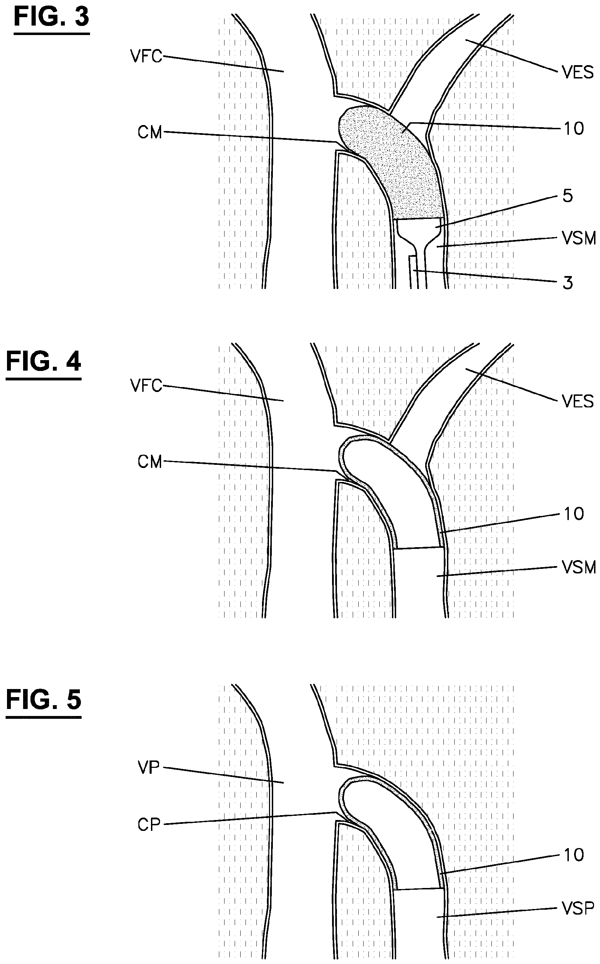

Fig. 3 zeigt z.B. die Einmündung CM der Vena saphena magna VSM in die Vena femoralis communis VFC ("Crosse"). Weiter ist die Vena epigastrica superficialis VES dargestellt, die in die Vena saphena magna VSM mündet.

Bei der Anwendung wird der Führungsdraht 4 in der Vene VSM bis zur Crosse CM gestossen und dann die Vorrichtung gemäss Fig. 1 in die Vene VSM eingeführt, indem der zweite Kanal 3 über den Draht 4 geschoben wird. Befindet sich der Ballon 5 zusammen mit der Hülle 10 an der gewünschten Stelle, wird der Führungsdraht 4 entfernt. Dann wird der Ballon 5 inflatiert. Entsprechend weitet sich die Hülle 10, vgl. Fig. 3. Die Hülle 10 kommt schliesslich in Kontakt mit der Venenwandung und bleibt dort haften. Der Ballon 10 wird entleert und aus der Vene VSM entnommen. Die Hülle 10 ist nun platziert, so wie in Fig. 4 gezeigt.

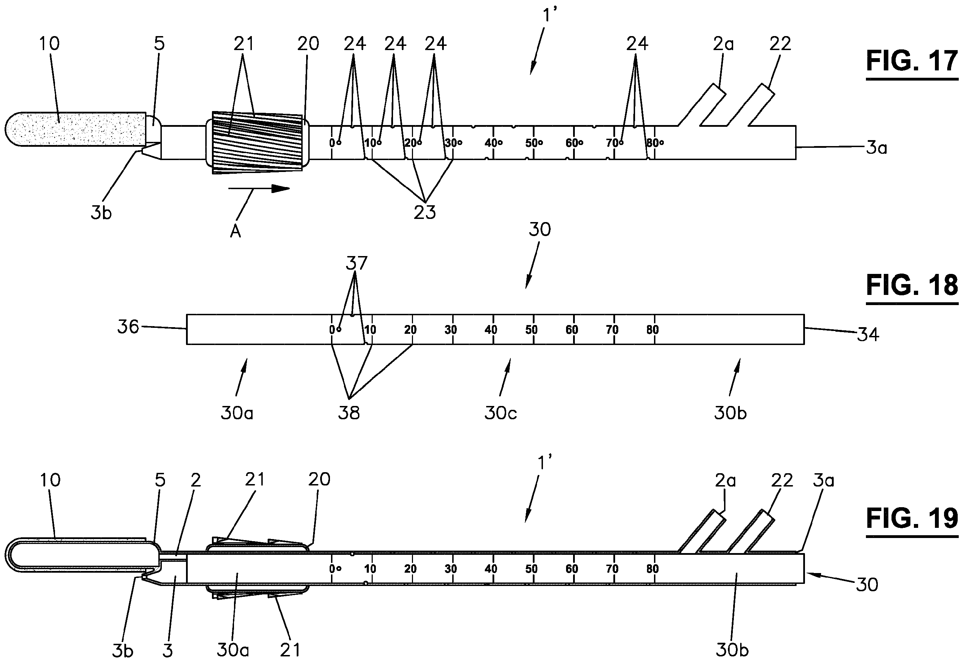

Die Crosse CM ist nun verschlossen, und die Vene VSM kann weiter behandelt werden, z.B. so wie unten im Zusammenhang mit den Fig. 17-19 erläutert.

Fig. 5 zeigt eine anderes Beispiel, bei welchem die Hülle 10 analog wie beim Beispiel gemäss Fig. 4 so gesetzt worden ist, dass die Crosse CP der Vena saphena parva VSP, die in die Vena poplitea VP mündet, verschlossen wird.

Das Anhaften der Hülle 10 an der Venenwandung ist durch mindestens ein Haftmittel erzielbar. Dieses ist so ausgelegt, dass zusammen mit der Hülle 10 ein nicht-starres Gebilde entsteht, welches schmerzfreie Bewegungen ermöglicht.

Ein Haftmittel ist in vielfältiger Weise bereitstellbar:
Die Hülle 10 kann z.B. eine Aussenschicht aufweisen, die beim Kontakt mit der Venenwand dort haften bleibt. Die Aussenschicht ist vorzugsweise aufweitbar und kann z.B. Kollagen enthalten.

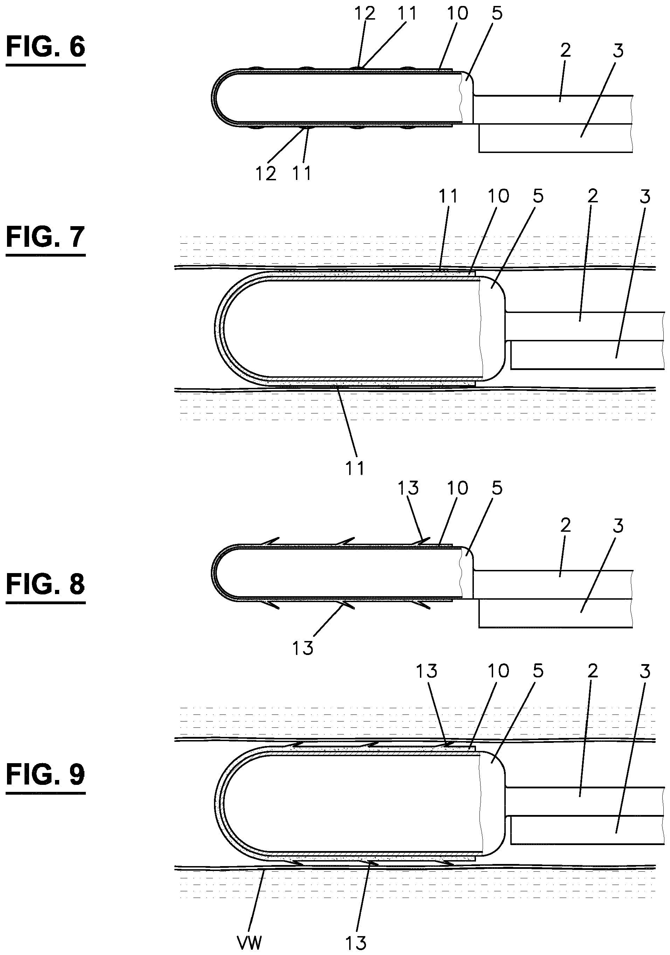

Fig. 6 zeigt ein Beispiel, bei welchem die Hülle 10 umlaufend auf ihrer Oberfläche mehrere Reservoire aufweist, die einen körperverträglichen Klebstoff 11 enthalten und durch ein Häutchen 12 abgedeckt sind. Beim Inflatieren des Ballons 5 platzen die Häutchen 12 und der Klebstoff 11 wird freigegeben. Ist der Ballon 5 genügend geweitet, so wie in Fig. 7 dargestellt, kommt der Klebstoff 11 in Kontakt mit der Venenwand, so dass die Hülle 10 daran festgeklebt wird. Als Klebstoff 11 eignet sich ein Gewebekleber, z.B. ein solcher basierend auf Cyanoacrylat.

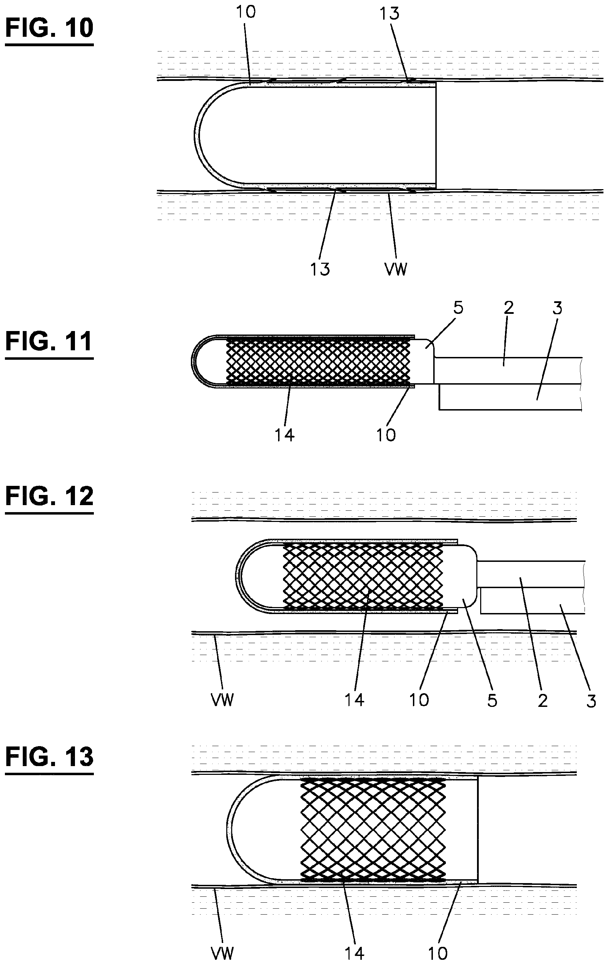

Fig. 8 zeigt ein Beispiel, bei welchem die Hülle 10 aussenseitig und umlaufend mit Hakenelementen 13 versehen ist, die zum Eingriff in die Venenwand eingerichtet sind. Beim Aufweiten des Ballons 9, vgl. Fig. 9, kommen die Hakenelemente 13 in Kontakt mit der Venenwand VW und werden schliesslich darin verankert, vgl. Fig. 10.

Fig. 11 zeigt ein Beispiel, bei welchem die Hülle 10 mit einem röhrenförmigen Gittergerüst 14 versehen ist. Dieses kann z.B. ähnlich wie ein Stent ausgebildet sein, erstreckt sich aber hier nicht unbedingt über die ganze Länge der Hülle 10. Das Gittergerüst 14 weitet sich auf, wenn der Ballon 5 geweitet wird, und verbleibt beim Entfernen des Ballons 5 im aufgeweiteten Zustand. Hier ist das Gittergerüst 14 zwischen Ballon 5 und Hülle 10 angeordnet. Es kann auch aussenseitig der Hülle 10 angeordnet sein. Das Gittergerüst 14 kann an der Hülle 14 befestigt sein. Das Gittergerüst 14 ist aus einem körperverträglichen Material, z.B. Metall. Je nach Anwendungszweck ist das Material resorbierbar.

Bei der Anwendung wird der Ballon 5 zusammen den darauf angeordneten Elementen 10 und 14 an die gewünschte Stelle in der Vene gebracht und anschliessend inflatiert, vgl. Fig. 12, bis die Hülle 10 an der Venenwand VW anliegt. Der Ballon 5 wird entleert und herausgenommen. Die Hülle 10 ist nun an der Venenwand VW mittels des Gittergerüsts 14 fixiert, vgl. Fig. 13.

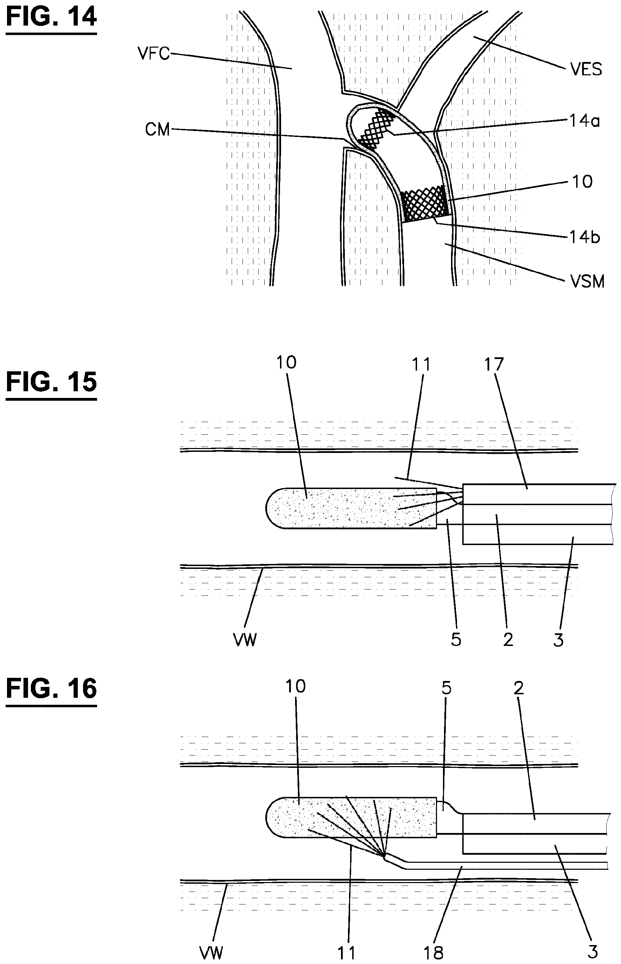

Als Alternative zu der Ausführungsform gemäss Fig. 11 ist es auch denkbar, zwei oder mehr Gittergerüste vorzusehen, welche entlang des Ballons 5 angeordnet sind. Z.B. ist es denkbar, zwei Gittergerüste vorzusehen, welche jeweils endseitig der Hülle 10 angeordnet sind, so dass dazwischen ein Abschnitt entsteht, welcher nur von der Hülle 10 gebildet wird. Fig. 14 zeigt ein Beispiel einer Hülle 10 mit zwei Gittergerüsten 14a, 14b, welche in der Crosse CM platziert worden ist. Das jeweilige Gittergerüst 14a, 14b ist an der Hülle 10 angeordnet, d.h. auf oder in der Hülle 10, und ist vorzugsweise daran befestigt.

Fig. 15 und 16 zeigen Varianten, bei welchen innerhalb der Vene ein Klebstoff 11 auf die Oberfläche der Hülle 10 appliziert werden kann, nachdem der Ballon 5 platziert, aber noch nicht geweitet ist. Der Klebstoff 11 wird abgegeben und verteilt sich über der Hülle 10. Anschliessend erfolgt die Inflation des Ballons 5 und die Hülle 10 wird so an die Venenwand VW geklebt.

Bei der Variante gemäss Fig. 15 ist am Katheter 2, 3 mindestens ein weiterer Kanal 17 angebracht, der fluidisch mit einem weiteren Anschluss am proximalen Ende des Katheters 2, 3 zum Einleiten des Klebstoffs 11 verbunden ist.

Bei der Variante gemäss Fig. 16 ist der weitere Kanal 18 separat vom Katheter 2, 3 ausgebildet. Der weitere Kanal 18 wird z.B. mittels einer zweiten Venenpunktion eingeführt.

Fig. 17-19 zeigen ein ergänztes Ausführungsbeispiel, bei welchem die Vorrichtung nebst dem Verschliessen einer Veneneinmündung eine Behandlung der Vene durch Applizieren eines Sklerosierungsmittels und durch Zerschneiden ermöglicht. Diese Behandlung ist im Patent EP3135206B1 des gleichen Anmelders beschrieben.

Der Katheter 1' ist ein Aussenkatheter und weist am distalen Ende den Ballon 5 mit der aufweitbaren Hülle 10 auf. Wie Fig. 19 zeigt, ist der Ballon 5 über einen ersten Kanal 2 fluidisch mit dem Anschluss 2a am proximalen Ende verbunden. Der Katheter 1' weist den zweiten Kanal 3 auf, welcher die Eingangsöffnung 3a mit der Ausgangsöffnung 3b verbindet und in welchen ein Führungsdraht aufnehmbar ist.

Auf dem Katheter 1' ist nachfolgend zum Ballon 5 ein weiterer Ballon 20 mit Schneidelementen 21 angeordnet. Der weitere Ballon 20 ist über einen weiteren (hier nicht dargestellten) Kanal fluidisch mit einem weiteren Anschluss 22 am proximalen Ende des Katheters 1' verbunden. So wie beim Ballon 5 und dem Anschluss 2a kann über den Anschluss 22 ein Fluid in den Ballon 20 gepumpt werden, um diesen zu Inflatieren.

Der Katheter 1' ist fenestriert. Zu diesem Zweck ist er zwischen dem Ballon 20 und den Anschlüssen 2a, 22 mit Seitenöffnungen 24 ("Katheterfenster") versehen. Diese sind um den Umfang des Katheters 1' herum verteilt angeordnet und über den Kanal 3 fluidisch mit der Eingangsöffnung 3a verbunden. Bei der Anwendung ist über die Seitenöffnungen 24 ein Sklerosierungsmittel in die zu behandelnde Vene injizierbar. Zwei benachbarte Seitenöffnungen 24 sind axial und radial versetzt zueinander angeordnet. Der Versatz erlaubt eine möglichst homogene Verteilung von Sklerosierungsmittel in der Vene.

Zwischen dem Ballon 20 und den Anschlüssen 2a, 22 sind auf dem Katheterschaft Markierungen 23 vorgesehen, die beispielsweise in regelmässigen Abständen angebracht sind und u.a. Angaben darüber liefern, wie weit der Katheter 1' in eine Vene eingeführt ist. In der Variante gemäss Fig. 17 sind Markierungen 23 in Form von Strichen und Zahlen 0, 10, 20, 30, ..., 80 zu sehen. Natürlich sind auch andere Arten von Markierungen möglich.

Die Seitenöffnungen 24 sind in Gruppen angeordnet, so dass der mit den Seitenöffnungen 24 versehene Teil des Katheters 1' in Abschnitte unterteilt ist, die jeweils die gleiche Anordnung an Seitenöffnungen 24 aufweist. Im Beispiel gemäss Fig. 17 ist z.B. zwischen dem Abschnitt 0 bis 10 eine Gruppe mit drei Seitenöffnungen zu sehen. Dieselbe Anordnung an Seitenöffnungen wiederholt sich im dem jeweiligen nachfolgenden Abschnitt 10 bis 20, 20 bis 30, etc. Die Seitenöffnungen 24 in der jeweiligen Gruppe sind radial um einen Winkel versetzt angeordnet. Beim Beispiel mit drei Seitenöffnungen kann dieser Winkel 120 Grad betragen. Es ist jedoch auch eine ungleichmässige radiale Verteilung denkbar. Im Weiteren kann die Anzahl der Seitenöffnungen 24 pro Gruppe bzw. Abschnitt anders als in Fig. 17 gezeigt sein und eins, zwei oder mehr betragen. Da die Seitenöffnungen 24 durch die Aussenwandung des Katheters 1' hindurchreichen, sind Anzahl und Anordnung so gewählt, dass genügend Platz bleibt, um die Kanäle von den Anschlüssen 2a und 22 zu den Ballonen 5 und 20 zu vorsehen zu können.

Über die Eingangsöffnung 3a des Katheters 1' ist ein zweiter Katheter 30 (im Folgenden auch "Innenkatheter") einführbar, wie er in Fig. 18 gezeigt ist. Der Katheter 30 weist einen distalen Endabschnitt 30a und einen proximalen Endabschnitt 30b mit einem Anschluss 34 auf. Der distale Endabschnitt 30a ist durch eine geschlossene Wandung gebildet. Insbesondere ist im Gegensatz zur Endöffnung 3b im Aussenkatheter 1' das Ende 36 des Endabschnitts 30a frei von einer Endöffnung. Das geschlossene Ende 36 des Innenkatheters 30 erlaubt ein Verschliessen des Lumen an der Spitze des Aussenkatheters 1', wodurch bei der Anwendung von Sklerosierungsmittel ein Wegfliessen durch die Ausgangsöffnung 3b verhindert wird.

Zwischen den beiden Endabschnitten 30a und 30b weist der Katheter 30 einen Zwischenteil 30c auf, der einen Innenkanal ("Lumen") aufweist und der mit Seitenöffnungen 37 versehen ist. Diese sind über den Innenkanal fluidisch mit dem Anschluss 34 verbunden. Der Katheter 30 ist anschliessend zum distalen geschlossenen Endabschnitt 30a lediglich auf einem Teilabschnitt mit Seitenöffnungen 37 versehen, während der übrige Katheterschaft keine Öffnungen aufweist. Es ist somit im Gegensatz zum Aussenkatheter 1' nur eine einzelne Gruppe an Seitenöffnungen 37 vorgesehen. Vorzugsweise entspricht die Anzahl und/oder Anordnung der Seitenöffnungen 37 der Anzahl bzw. Anordnung der ersten Gruppe an Seitenöffnungen 24 beim Aussenkatheter 1'. Im Beispiel gemäss Fig. 18 sind drei Seitenöffnungen 37 zu sehen, die radial und axial versetzt zueinander angeordnet sind ähnlich wie die Seitenöffnungen 24 in einer Gruppe beim Aussenkatheter 1' gemäss Fig. 17. Je nach Auslegung kann die Anzahl der Seitenöffnungen 37 eins, zwei oder mehr betragen.

Entlang des Zwischenteils 30c des Katheters 30 sind Markierungen 38 vorgesehen, die beispielsweise in regelmässigen Abständen angebracht sind und u.a. Angaben darüber liefern, wie weit der Katheter 30 in den Aussenkatheter 1' eingeführt ist. Als Markierungen 38 dienen z.B. Striche, Zahlen, etc.

Die Aussenform des Innenkatheters 30 ist so gestaltet, dass im Aussenkatheter 1' Platz für den Kanal 2 zum Ballon 5 sowie für den Kanal zum Ballon 20 vorgesehen werden kann.

Bei der Anwendung wird der Innenkatheter 30 im Aussenkatheter 1' aufgenommen und dann abschnittsweise zurückgezogen. Dabei befinden sich die Seitenöffnungen 38 zuerst in der Nähe der Seitenöffnungen 24 der ersten Gruppe, anschliessend bei den Seitenöffnungen 24 der zweiten Gruppe, usw. Der verschlossene Endabschnitt 30a des Innenkatheters 30 dichtet dabei den Kanal 3 des Aussenkatheters 1' zwischen den Enden 3b und 36 ab. Über den Anschluss 34 und die Seitenöffnungen 37 und 24 ist somit ein Sklerosierungsmittel abschnittsweise in die zu behandelnde Vene einleitbar.

Der Ballon 20 ist in den Fig. 17 und 19 in einem leicht inflatierten Zustand dargestellt ist. Im Folgenden wird als "axial" die Richtung bezeichnet, in welcher die Achse A verläuft, entlang welcher sich der Aussenkatheter 1' von der Ausgangsöffnung 3b zur Eingangsöffnung 3a erstreckt, während "radial" quer zur Achse A ist.

Der Ballon 20 ist ein "Cutting Balloon" und weist zu diesem Zweck ein oder mehrere Schneidelemente 21 ("Klingen") auf. Ein jeweiliges Schneidelement 32 verläuft in axialer Richtung A gesehen nicht gerade.

Es sind vielfältige Formen an Verläufen denkbar. Beispielsweise kann die Schnittkante eines Schneidelements so gekrümmt sein, dass sie sich um die Achse A windet, z.B. schraubenförmig. Es ist auch denkbar, dass in Richtung der Achse A gesehen die Schnittkante einen Abschnitt mit einem geraden axialen Verlauf aufweist, der über einen gekrümmten Zwischenabschnitt wieder in einen geraden Abschnitt übergeht. Es ist auch denkbar, nur ein einzelnes Schneidelement 3 vorzusehen, welches um die Achse A verläuft.

Der ungerade Verlauf eines Schneidelements 3 führt dazu, dass gesehen in der axialen Richtung A die Enden eines Schneidelements 3 um einen Winkel radial versetzt angeordnet sind, der grösser als 0 Grad ist. Bevorzugt beträgt der Winkel mindestens 10 Grad und besonders bevorzugt mindestens 20 Grad. Im Weiteren kann der Verlauf so sein, dass besagter Winkel weniger als 360 Grad ist. Bevorzugt beträgt er höchstens 180 Grad und besonders bevorzugt höchstens 90 Grad.

Die maximale Ausdehnung eines Schneidelements 21 quer zur Achse A kann ebenfalls veränderlich sein, indem die Schneidkante auf einer Höhe verläuft, die in Richtung zum Anschluss 3a hin abnimmt. Im vorliegenden Ausführungsbeispiel ist das Schneidelement 21 keilförmig ausgebildet. Die maximale Höhe H eines Schneidelements 21 ist typischerweise im Bereich von 0.5 - 1.5 mm.

Der Ballon 21 erstreckt sich axial auf einer Länge, welche typischerweise im Bereich von 5 - 30 mm liegt.

Die sich axial bzw. radial veränderliche Formgebung eines Schneidelements 21 erlaubt eine umfassende mechanische Einwirkung auf eine Veneninnenwand, wenn der in die Vene eingeführte Katheter 1' wieder herausgezogen wird. Dabei graben sich die sich verjüngenden Schneidkanten der Schneidelemente 21 ähnlich eines Pflugs allmählich in die Veneninnenwand ein. Es wird daher eine abrupte mechanische Einwirkung vermieden, so dass eine schmerzfreiere Behandlung möglich ist, die unter Umständen auch ohne örtliche Betäubung in Form einer Tumeszenzanästhesie erfolgen kann. Dies ist z.B. dann der Fall, wenn Aethoxysklerol® als Sklerosierungsmittel eingesetzt wird, das ja auch ein Lokalanästhetikum ist.

Die Vorrichtung gemäss Fig. 17-19 ist z.B. wie folgt einsetzbar:
Der Katheter 1' wird gegebenenfalls mittels Führungsdraht in die Stammvene VSM eingeführt und die Hülle 10 durch In- und Deflatieren des Ballons 5 platziert, so dass die Crosse CM verschlossen ist, vgl. Fig. 4.

Anschliessend wird der Ballon 20 inflatiert. Der Innenkatheter 20 wird in den Kanal 3 des Aussenkatheters 1' einschoben, sofern er nicht schon zusammen mit dem Katheter 1' eingeführt worden ist im Falle, dass kein Führungsdraht verwendet wurde.

Mittels einer am Anschluss 34 angebrachten Spritze wird über den Innenkatheter 20 das Sklerosierungsmittel zugeführt. Der Innenkatheter 30 wird in z.B. 10 cm-Schritten herausgezogen. Pro 10 cm wird jeweils Sklerosierungsmittel appliziert und eine bestimmte Zeit, z.B. ca. 1 Minute, gewartet, bevor der nächste Abschnitt behandelt wird. Es wird so weiterverfahren, bis der gesamte Innenkatheter 30 entfernt ist.

Die Einwirkung des Sklerosierungsmittels hat zu einem Vasospasmus geführt. Zudem sollte auch die Schmerzempfindlichkeit vermindert sein. Nun wird der Katheter 1 mit dem inflatierten Ballon 20 langsam zurückgezogen. Durch die mechanische Einwirkung eines Schneidelements 21 auf dem Ballon 20 wird die Intima und Media der Vene zerstört. Die spezielle Formgebung eines Schneidelements 21 erlaubt eine wirksame Zerstörung der Veneninnenwand, die beim Zurückziehen des Ballons 20 in multiple Fragmente zerschnitten wird.

### Zweites Ausführungsbeispiel

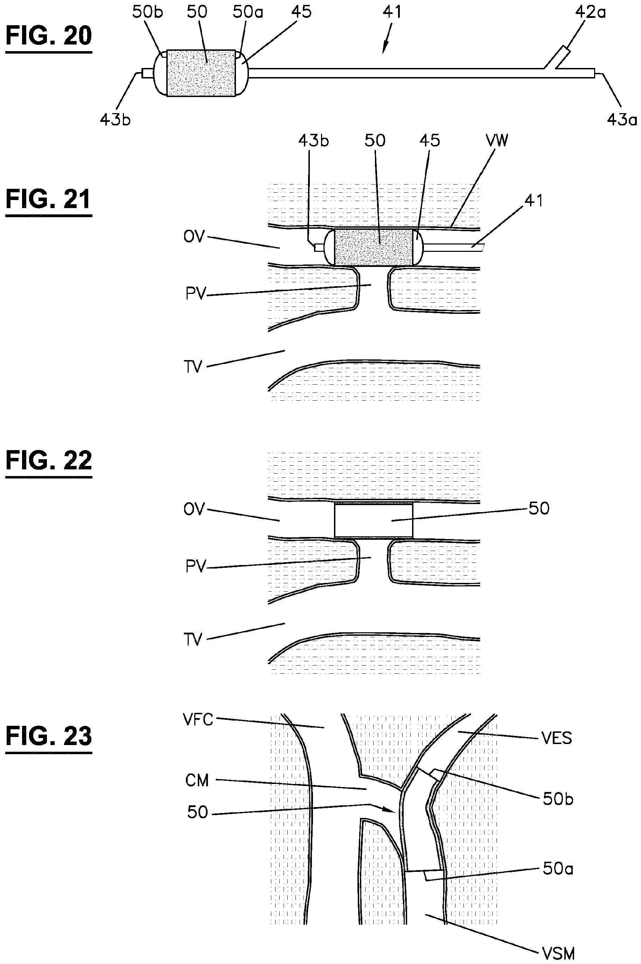

Beim in Fig. 20 gezeigten Ausführungsbeispiel umfasst die Vorrichtung einen Katheter 41, welcher einen ersten Kanal aufweist, der eine Eingangsöffnung 43a mit der Ausgangsöffnung 43b verbindet und der zur Aufnahme eines Führungsdrahtes 4 (vgl. Fig. 2) ausgelegt ist. Am distalen Ende des Katheters 41 ist ein Ballon 45 angeordnet. Dieser verläuft um die Aussenwandung des Katheters 41 herum, so dass der erste Kanal sich durch den Ballon 45 hindurch erstrecken kann und sich die Ausgangsöffnung 43b axial versetzt zum Ballon 45 befindet. Der Ballon 45 ist über einen zweiten Kanal mit einem Anschluss 42a am proximalen Ende des Katheters 41 fluidisch verbunden. Über den zweiten Kanal ist ein Fluid förderbar, um den Ballon 45 in- und deflatieren zu können.

Auf dem Ballon 45 ist eine aufweitbare Hülle 50 angeordnet. Diese ist wie die Hülle 10 beim ersten Ausführungsbeispiel als Membran ausgebildet und kann aus demselben Material bestehen.

Im Gegensatz zur Hülle 10 ist die Hülle 50 beidseitig an beiden Hüllenenden 50a, 50b offen und somit durchgehen röhrenförmig ausgestaltet.

Die Länge der Hülle 50 (Distanz zwischen den Enden 50a und 50b) ist im nicht-aufgeweiteten Zustand typischerweise kleiner als 10 cm.

Zur Fixation der Hülle 50 an einer Venenwand ist mindestens ein Haftmittel vorgesehen, das so ausgestaltet sein kann wie beim ersten Ausführungsbeispiel: Vorsehen einer haftenden Aussenschicht auf der Hülle 50, Vorsehen von Klebstoff 12 mittels Reservoir 11 und/oder durch einen weiteren Kanal 17, 18, Vorsehen von Hakenelementen 13, Vorsehen eines oder mehrerer aufweitbaren Gittergerüsten 14, etc.

Die Vorrichtung gemäss Fig. 20 ist z.B. anwendbar, um eine Veneneinmündung in eine Perforansvene zu verschliessen.

Fig. 21 zeigt schematisch eine Perforansvene PV, welche eine oberflächliche Vene OV mit einer tiefen Vene TV verbindet. Die Perforansvene PV ist insuffizient und soll still gelegt werden. Dazu wird wie folgt vorgegangen:
Nachdem der Führungsdraht 4 unter Ultraschallkontrolle über die Vene OV in den Bereich der zu behandelnden Perforansvene PV gestossen worden ist, wird der Katheter 41 entlang des Führungsdrahts 4 soweit vorgeschoben, bis der Ballon 45 zusammen mit der Hülle 50 die Perforansvene PV abdeckt. Der Führungsdraht 4 wird entfernt und der Ballon 45 wird inflatiert, bis die Hülle 50 die Venenwandung VW kontaktiert, vgl. Fig. 21. Aufgrund des Haftmittels bleibt die Hülle 50 an der Venenwandung VW haften. Der Ballon 45 wird entleert und durch Herausziehen des Katheters 41 entfernt. Die Veneneinmündung in die Perforansvene PV ist nun mittels der Hülle 50 verschlossen, vgl. Fig. 22.

Die Vorrichtung gemäss Fig. 20 ist auch für die Behandlung der Magnacrosse CM einsetzbar. Fig. 23 zeigt die Situation, bei welcher die beidseitig offene Hülle 50 so platziert worden ist, dass das offene Ende 50b Teil in die Vena epigastrica superficialis VES hineinreicht, wodurch die Verbindung zur Vena femoralis communis VFC abgedichtet ist.

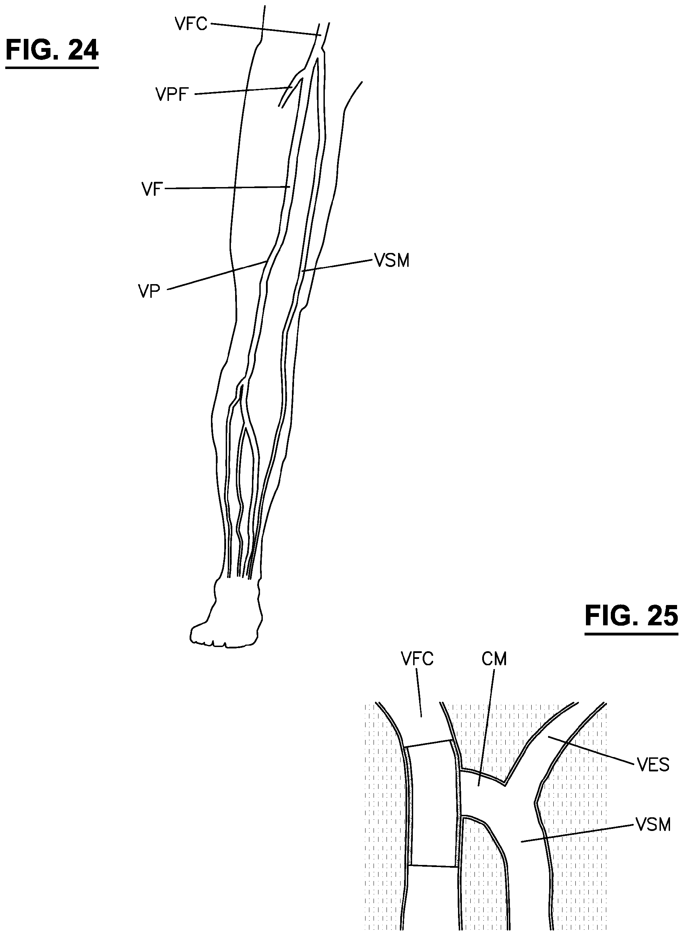

Als weitere Anwendungsvariante ist es möglich, die Hülle 50 so zu setzen, dass sie die Vena saphena magna VSM bzw. ihre Crosse CM von der Vena femoralis communis VFC aus verschliesst, vgl. Fig. 25.

Fig. 24 zeigt einige Beinvenen, namentlich Vena poplitea VP, Vena femoralis VF, Vena femoralis communis VFC, Vena profunda femoris VPF und Vena saphena magna VSM.

Der Katheter 41 mit dem Ballon 45 und der Hülle 50 wird über eine Punktion in der Kniekehle in die Vena poplitea VP und damit ins tiefe Beinvenensystem eingebracht. Ballon 45 und Hülle 50 werden über die Vena femoralis VF bis in die Vena femoralis communis VPF hochgeschoben und die Hülle bei der Magnacrosse CM platziert.

Die hier beschriebenen Vorrichtungen können für einen Einweggebrauch ausgelegt werden und sind in steriler Form in Verpackungen bereitgestellt.

Aus der vorangehenden Beschreibung sind dem Fachmann zahlreiche Abwandlungen zugänglich, ohne den Schutzbereich der Erfindung zu verlassen, der durch die Ansprüche definiert ist.

Die Schneideinrichtung 20, 21 und/oder die Seitenöffnungen 24 in der Variante gemäss Fig. 17 kann/können auch auf einem separat ausgebildeten Katheter ausgebildet sein, der in die Vene einchiebbar ist, nachdem die Hülle 10, 50 platziert und die Vorrichtung gemäss Fig. 1 bzw. 20 entfernt worden ist.

## Patentansprüche

1. Vorrichtung zum Verschliessen einer Veneneinmündung bei der Behandlung der Varikose, umfassend
einen in ein Venensystem einführbaren Katheter (1, 1', 41), an dessen distalen Ende ein aufweitbarer Ballon (5, 45) angeordnet ist,
eine Hülle (10, 50), welche umlaufend auf dem Ballon angeordnet ist und mittels diesem aufweitbar ist, wobei sich die Hülle von einem proximalen Hüllenende (10a, 50a) zu einem distalen Hüllenende (10b, 50b) erstreckt und wobei das proximale Hüllenende offen ausgestaltet ist, und
mindestens ein Haftmittel (11-14, 17, 18) zum Einwirken auf die aufgeweitete Hülle derart, dass sie zum Verschliessen der Veneneinmündung an einer Venenwand anhaftet.

2. Vorrichtung nach Anspruch 1, wobei das mindestens eine Haftmittel (11-14, 17, 18) mindestens eines der folgenden Elemente A1-A7 umfasst:
A1) die Hülle (10, 50) ist mit einer haftenden Aussenschicht versehen,
A2) die Hülle umfasst mindestens ein Reservoir (12) mit einem Klebstoff (11), wobei vorzugsweise das Reservoir beim Aufweiten der Hülle aufplatzt und den Klebstoff freigibt,
A3) die Hülle weist mindestens ein Hakenelement (13) auf, welches zum Eingriff in die Venenwand ausgestaltet ist,
A4) es ist mindestens ein aufweitbares Gittergerüst (14, 14a, 14b) vorgesehen zum Einwirken auf die Hülle derart, dass sie an der Venenwand haftet, vorzugweise ist das mindestens eine Gittergerüst rohrförmig ausgebildet und/oder vorzugsweise so ausgebildet, dass es mit einem wirksamen Abstand zum distalen und/oder proximalen Hüllenende (10a, 10b, 50a, 50b) angeordnet ist, besonders bevorzugt sind zwei aufweitbare Gittergerüste (14a, 14b) vorgesehen, welche mit einem wirksamen Abstand dazwischen an der Hülle (10) angeordnet sind,
A5) es ist mindestens ein Kanal (17, 18) vorgesehen, durch welchen ein Klebstoff (11) leitbar ist, um ihn auf der Hülle auftragen zu können, vorzugsweise ist der Kanal Teil eines weiteren Katheters, der fest mit dem Katheter (1) verbunden oder der lose von diesem ausgestaltet ist,
A6) das mindestens eine Haftmittel wirkt zwischen der Hülle und der Venenwand,
A7) das Einwirken des mindestens einen Haftmittels ist derart, dass beim Anhaften der Hülle an der Venenwand das proximale Hüllenende (10a, 50a) offen bleibt.

3. Vorrichtung nach einem der vorangehenden Ansprüche, wobei die Hülle mindestens eines der folgenden Merkmale B1-B5 aufweist:
B1) die Hülle (10, 50) ist am distalen Hüllenende (10b, 50b) geschlossen oder offen ausgebildet,
B2) die Distanz zwischen dem proximalen (10a, 50a) und distalen Hüllenende (10b, 50b) ist im nicht-aufgeweiteten Zustand der Hülle kleiner als 10 cm, vorzugsweise kleiner als 8 cm, besonders bevorzugt ist die Distanz im Bereich von 4-7 cm,
B3) die Hülle auf dem Ballon (5, 45) weist im aufgeweiteten Zustand eine maximale Breite von höchstens 2 cm auf, bevorzugt liegt die maximale Breite im Bereich von 0.5-1.5 cm,
B4) die Hülle auf dem Ballon weist im nicht-aufgeweiteten Zustand im Mittel eine Dicke auf, die höchstens 1 mm beträgt, vorzugsweise höchstens 0.7 mm,
B5) die Hülle ist aus einem biologischen oder synthetischen Material, vorzugsweise ist die Hülle aus Rinderperikard.

4. Vorrichtung nach einem der vorangehenden Ansprüche, wobei der Katheter (1, 1', 41) einen ersten Kanal (2) aufweist, der einen Anschluss (2a) am proximalen Ende des Katheters fluidisch mit dem Ballon (5, 45) verbindet, und einen zweiten Kanal (3), welcher sich von einer Eingangsöffnung (3a, 43a) am proximalen Ende des Katheters zu einer Ausgangsöffnung (3b, 43b) am distalen Ende des Katheters erstreckt und zur Aufnahme eines Führungsdrahtes (4) dient.

5. Vorrichtung nach Anspruch 4, wobei die Ausgangsöffnung (3b) radial zum Ballon (5) versetzt angeordnet ist, so dass der Ballon mit der Hülle (10) seitlich zum im zweiten Kanal (3) aufgenommen Führungsdraht (4) anordbar ist,
oder wobei die Ausgangsöffnung (43b) axial zum Ballon (45) versetzt angeordnet ist, so dass der im zweiten Kanal aufgenommene Führungsdraht durch den Ballon hindurch verlaufen kann.

6. Vorrichtung nach einem der vorangehenden Ansprüche, welche zur Behandlung einer zur Veneneinmündung führenden Vene eine Schneideinrichtung (20, 21) zum Zerschneiden zumindest eines Teils der Innenwand der Vene und/oder einen Zufuhrkanal (3) mit Seitenöffnungen (24) aufweist, durch welche hindurch ein Sklerosierungsmittel in die Vene einleitbar ist.

7. Vorrichtung nach Anspruch 6, wobei die Schneideinrichtung (20, 21) auf dem Katheter (1') oder auf einem separat davon ausgebildeten weiteren Katheter angeordnet ist, vorzugsweise weist die Schneideinrichtung einen aufweitbaren Schneidballon (20) mit mindestens einem Schneidelement (21) auf.

8. Vorrichtung nach Anspruch 6 oder 7, wobei der Katheter (1') oder ein davon separat ausgebildeter weiterer Katheter den Zufuhrkanal (3) mit den Seitenöffnungen (24) aufweist.

9. Vorrichtung nach einem der Ansprüche 6-8, weiter umfassend einen Innenkatheter (30), welcher in den Katheter (1') bzw. den weiteren Katheter einfügbar ist und in welchen das Sklerosierungsmittel einleitbar ist, wobei der Innenkatheter Seitenöffnungen (37) aufweist, vorzugsweise ist der Innenkatheter am distalen Ende (36) verschlossen ausgebildet und/oder vorzugsweise ist Anzahl der Seitenöffnungen (37) am Innenkatheter kleiner als die Anzahl der Seitenöffnungen (24) im Zufuhrkanal (3).

10. Vorrichtung nach einem der vorangehenden Ansprüche, welche mindestens eine der folgenden Komponenten C1-C2 aufweist
C1) einen Führungsdraht (4), aufweichen der Katheter (1, 1', 41) aufschiebbar ist,
C2) einen Klebstoff (11), mittels welchem die Hülle (10, 50) an der Venenwand anklebbar ist.
